# EUROPÄISCHE PATENTSCHRIFT

(11) **EP 2 000 123 B1**
(45) Veröffentlichungstag und Bekanntmachung des Hinweises auf die Patenterteilung: **09.12.2015**
(21) Anmeldenummer: 08154506.3
(22) Anmeldetag: 15.04.2008
(51) Int. Cl.: A61K 8/41, A61K 8/73, A61K 8/81, A61K 8/86, A61K 8/92, A61Q 17/04, A61Q 19/00, A61Q 19/04, A61Q 17/00

(54) **Stabile, niedrigviskose kosmetische Zusammensetzungen enthaltend Esterquats und/oder Dialkylquats**
Stable, low viscosity cosmetic compounds containing esterquat and/or dialkylquat
Compositions cosmétiques stables et peu visqueuses comprenant des esters quaternaires et/ou des dialkyles quaternaires

(30) Priorität: 08.06.2007 DE 102007027030
(43) Veröffentlichungstag der Anmeldung: 10.12.2008
(73) Patentinhaber: Deb IP Limited, Denby, Derbyshire DE5 8JZ (GB)
(72) Erfinder: Jenni, Klaus, 45357, Essen (DE); Mathiak, Ralf, 45968, Gladbeck (DE); Veeger, Marcel, 47574, Goch (DE); Meyer, Jürgen, 48143, Münster (DE)
(74) Vertreter: Elsy, David

(56) Entgegenhaltungen:
- WO-A-01/00158
- WO-A-03/005985
- DE-A1- 19 652 300
- US-A- 5 013 763
- US-A1- 2005 288 198

## Beschreibung

Die vorliegende Erfindung betrifft kosmetische Zusammensetzungen zum Auftragen auf die Haut, die Esterquats enthalten.

Unter dem Begriff "Esterquats" werden im Allgemeinen quaternisierte Fettsäurealkanolaminester sowie Salze derselben verstanden. Es handelt sich hierbei um kationische Tenside, die im Allgemeinen eine geringe Toxizität und gute biologische Abbaubarkeit aufweisen. Sie weisen folgende allgemeine Strukturformel auf: 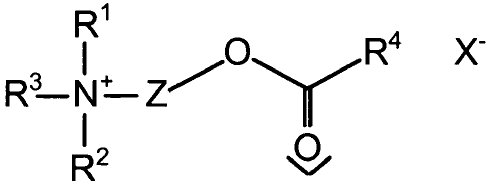
- R⁴: ist dabei ein Acyl-Rest, während Z eine Alkylengruppe darstellt. Die Gruppe Z-OOCR⁴ kann damit auch als Fettsäure-Alkanolestergruppe bezeichnet werden. Die Reste R¹ bis R³ können hingegen Wasserstoff- oder Alkylgruppen bzw. Ester aus Alkylengruppen und Acylresten sein,
- X⁻: ist ein Anion und kann insbesondere Chlorid, Bromid, Methosulfat, Nitrat, Acetat, Phosphat oder Tosylat sein.

Esterquats weisen durchweg kationische Eigenschaften auf und werden daher auch den kationischen Tensiden zugerechnet. Aufgrund ihrer besonderen, konditionierenden Eigenschaften werden sie insbesondere in Weichspülern eingesetzt. Zudem erzeugen sie ein angenehmes Haar- und Hautgefühl und kommen daher auch als Emulgatoren oder Conditioner in kosmetischen Zusammensetzungen zum Einsatz. Überdies verleihen sie solchen Zusammensetzungen unter bestimmten Bedingungen wasserfeste Eigenschaften.

Es handelt sich bei Esterquats um an sich bekannte Stoffe, die man nach den einschlägigen Methoden der präparativen organischen Chemie darstellen kann.

Die Herstellung von Esterquats wird beispielsweise in der WO-A-91/01295 beschrieben, nach der man Triethanolamin in Gegenwart von unterphosphoriger Säure mit Fettsäuren partiell verestert, Luft durchleitet und anschließend mit Dimethylsulfat oder Ethylenoxid quaternisiert. Auch aus der Deutschen Patentschrift DE-C-43 08 794 ist ein Verfahren zur Herstellung fester Esterquats bekannt, bei dem man die Quaternierung von Triethanolaminestern in Gegenwart von geeigneten Fettalkoholen durchführt. Bei einem weiteren bekannten Verfahren zur Herstellung von Esterquats kann sowohl von Fettsäuren, als auch den entsprechenden Triglyceriden in Abmischung mit Dicarbonsäuren ausgegangen werden. Ein solches Herstellungsverfahren ist in der europäischen Patentschrift EP-B-0 750 606 beschrieben. Die Verwendung von Esterquats in kosmetischen Zusammensetzungen ist z.B. aus der DE-A-198 51 451, der EP-B-1 239 828, der EP-A-1 250 906 und der EP-B-1 254 653 bekannt.

Problematisch ist, dass in kosmetischen Zusammensetzungen enthaltene Esterquats in den Konzentrationen, die zur Erzielung der oben genannten Effekte mindestens erforderlich sind, insbesondere dann, wenn die Zusammensetzung außerdem Öle oder ölartige Bestandteile aufweist, eine hohe Viskosität der jeweiligen kosmetischen Zusammensetzung hervorrufen. Es hat sich hierbei außerdem herausgestellt, dass niedrigviskose Formulierungen, die die genannten Esterquats enthalten, ohne weitere Zusätze nicht Langzeit-lagerstabil sind.

Nun erfreuen sich gerade in jüngerer Zeit kosmetische Zusammensetzungen mit niedrigen Viskositäten einer immer größer werdenden Beliebtheit. Sie lassen sich leicht und schnell auf der Haut verteilen und vermitteln dem Verbraucher den Eindruck, dass sie schnell in die Haut einziehen. Hinzu kommt, dass sich niedrigviskose Hauptpflegemittel besonders gut mit den immer beliebter werdenden Pumpsprays auf die Haut auftragen und anschließend verstreichen lassen.

Bei den kosmetischen Zusammensetzungen der eingangs genannten Art lässt sich auf Öle oder ölartige Bestandteile nicht verzichten, da diese hautpflegende Eigenschaften aufweisen. In einigen Fällen kommen ölartigen Bestandteilen auch noch andere Aufgaben zu, so z.B. bei Sonnenschutzmitteln, die unter die Definition der kosmetischen Zusammensetzungen zum Auftragen auf die Haut im Sinne der vorliegenden Erfindung fallen und bei denen häufig ölartige Sonnenschutzfilter verwendet werden.

Aufgabe der vorliegenden Erfindung ist es daher, kosmetische Zusammensetzungen zum Auftragen auf die Haut enthaltend Esterquats bereit zu stellen, die niedrigviskose Eigenschaften aufweisen und Langzeit-lagerstabil sind.

Diese Aufgabe wird mit den Merkmalen des vorgelegten Hauptanspruchs gelöst.

Die Unteransprüche geben bevorzugte Ausführungsformen an. Dabei ist zu beachten, dass die genannten Bereichsangaben durchweg einschließlich der jeweiligen Grenzwerte zu verstehen sind. Hinzu kommt, dass sich die Angaben zu Viskositäten durchweg auf Raumtemperatur beziehen.

Demgemäß ist eine kosmetische Zusammensetzung zum Auftragen auf die Haut vorgesehen, aufweisend
a) 0,05 bis 5,0 Gew.-% eines Polyethylenglykol- bzw. Polypropylenglykolderivats, wobei dieses ausgewählt ist aus einem Polyethylenglykoldiester mit einem Molekulargewicht im Bereich zwischen 1.500 und 12.000 Dalton, einem Polypropylenglykoldiester oder einem Polyethylen/Polypropylenglykoldiester,
b) 10 bis 40 Gew.-% Öle und/oder ölartige Bestandteile (Ölphase), sowie
c) 0,2 bis 15 Gew.-% eines Esterquats.

Neben den Komponenten a), b) und c) enthält die Zusammensetzung vorzugsweise Wasser, bevorzugt soviel Wasser, dass sich die Summe der Komponenten zu 100 Gew.-% ergänzt.

Die Polyethylenglykol- bzw. Polypropylenglykolderivate fungieren in kosmetischen Zusammensetzungen bekanntermaßen als Verdickungsmittel.

So wird z.B. das von der Firma Goldschmidt GmbH unter dem Handelsnamen REWOPAL PEG 6000 DS angebotene Polyethylenglykoldistearat als Verdickungsmittel für Shampoos spezifiziert.

Hintergrund der Erfindung ist, dass die Erfinder festgestellt haben, dass bei Verwendung von Esterquats als kationische Emulgatoren in niedrigviskosen Emulsionen die Emulsionen in der Regel eine nicht ausreichende Lagerstabilität aufweisen.

Dabei haben die Erfinder erstmals gefunden, dass die Hinzufügung von Polyethylenglykol- bzw. Polypropylenglykolderivaten in den erfindungsgemäßen Anteilen die Lagerstabilität solcher Emulsionen entscheidend verbessert, ohne zu der eigentlich zu erwartenden Viskositätserhöhung zu führen - obwohl letztere als Verdickungsmittel bekannt sind.

Vor diesem Hintergrund ist es überraschend und für den Fachmann nicht vorhersehbar gewesen, dass gerade die Kombination aus Esterquats, Ölen und ölartigen Bestandteilen sowie Polyethylenglykol- bzw. Polypropylenglykolderivaten in den erfindungsgemäß beanspruchten Anteilen zu einem Produkt führt, dessen Viskosität in dem erfindungsgemäß beanspruchten, für kosmetische Zusammensetzungen sehr niedrigen Bereich liegt.

Die Hinzufügung der Polyethylenglykol- bzw. Polypropylenglykolderivaten in den erfindungsgemäß beanspruchten Anteilen führt zu einem verdünnenden Effekt, obwohl der Fachmann einen verdickenden Effekt erwartet hätte.

Auf diese Weise ist es daher erstmals möglich, eine kosmetische Zusammensetzung bereit zu stellen, die die ein angenehmes Hautgefühl hervorrufenden Esterquats sowie hautpflegende Öle und ölartige Bestandteile enthält und gleichzeitig eine ausreichend niedrige Viskosität aufweist, um so dem Wunsch des Verbrauchers nach einem einfach und angenehm aufzutragenden Produkt nachzukommen.

Bevorzugt ist dabei vorgesehen, dass die Viskosität der erfindungsgemäßen kosmetischen Zusammensetzung im Bereich zwischen 50 und 12.000 mPas liegt. Besonders bevorzugt liegt die Viskosität im Bereich zwischen 50 und 1.000 mPas. Die beanspruchten bzw. bevorzugten Viskositätsbereiche entsprechen relativ dünnflüssigen Produkten. Handelsübliche Hautcreme weist hingegen eine Viskosität von etwa 15.000 bis 30.000 mPas auf. Unter dem Begriff "Esterquats" werden, wie oben bereits beschrieben, im Allgemeinen quaternisierte Alkanolamin-Fettsäureester verstanden. Erfindungsgemäß bevorzugt ist die Verwendung von wenigstens einem Esterquat, wobei Gemische bevorzugt sind, welche die nachstehende Formel I, Formel II und/oder Formel III aufweisen:

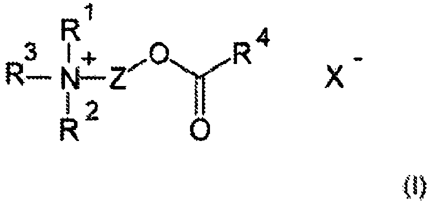

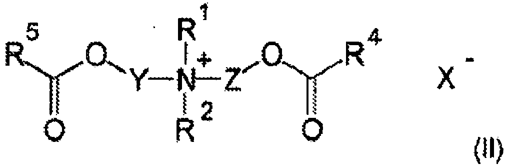

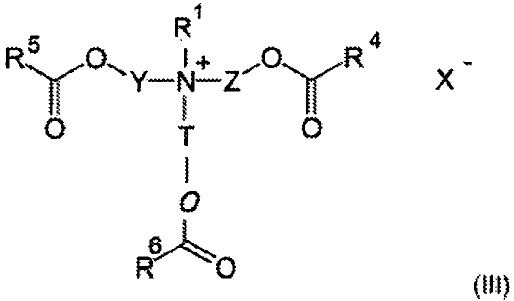

In den Formeln (I) bis (III) stehen die Reste R¹ bis R³ für Wasserstoff- oder Alkylgruppen bzw. Ester aus Alkylengruppen und Acylresten, die Reste R⁴, R⁵ und R⁶ für gleiche oder unterschiedliche Oleyl-, Talgalkyl-, Stearyl-, Palmityl-, Cocoyl-, Palmyl, Rapsalkyl-, Soyaalkylreste bzw. deren hydrierten Analoga und die Reste T, Y und Z für gleiche oder unterschiedliche zweiwertige Alkylenreste, vorzugsweise ausgewählt aus Ethylen-, Propylen-, Butylen-, Isopropylen- und Isobutylenresten. X ist ein Anion, und kann insbesondere Chlorid, Bromid, Methosulfat, Nitrat, Acetat, Phosphat oder Tosylat sein. Bevorzugt steht in den Formeln (I) bis (III) der Rest R¹ für einen Methylrest und die Reste R² und R³ für gleiche oder unterschiedliche Methyl-, Hydroxyethyl-, Hydroxypropyl-, Hydroxyisopropyl-, Hydroxybutyl- oder Hydroxyisobutylreste.

Gemäß einer besonders bevorzugten Ausführungsform der vorliegenden Erfindung können Esterquats verwendet werden, die ausgewählt sind aus der Gruppe umfassend:
Di-(oleylcarboxyethyl)-hydroxyethyl-methylammoniumsalz, Di-(talgcarboxyethyl)-hydroxyethyl-methylammoniumsalz, N,N-Di-(β-stearoylethyl)-N,N-dimethylammoniumsalz, N,N-Di-(β-palmitoyl-ethyl)-N,N-dimethylammoniumsalz, Dicocoylethyl-hydroxyethylammonium-methosulfat, Dipalmoylethyl-hydroxyethyl-ammonium-methosulfat, Dirapscarboxyethyl-hydroxyethylammonium-methosulfat, und/oder Disoyacarboxyethyl-hydroxyethylammonium-methosulfat und/oder deren hydrierte Analoga.

Gemäß der vorliegenden Erfindung können die Esterquats einzeln oder in Form von Gemischen verwendet werden. Im Hinblick auf die anwendungstechnischen Eigenschaften der Esterquats hat sich für Mischungen von Esterquats ein durchschnittlicher Veresterungsgrad von 1 bis 3, vorzugsweise 1,5 bis 2,5 und bevorzugt 1,7 bis 2,2 als besonders vorteilhaft herausgestellt. Weiterhin ist die Verwendung von Esterquats bevorzugt, bei denen es sich um technische Mischungen von Mono-, Di- und Triestern mit einem durchschnittlichen Veresterungsgrad von 1,5 bis 1,9 handelt. Zur Einstellung der gewünschten Jodzahl können die Esterquats nach gängigen Verfahren hydriert werden.

Ein besonders bevorzugtes Esterquat wird unter dem Handelsnamen Rewoquat WE 38 DPG von der Firma Goldschmidt vertrieben. Dieses Esterquat ist wie folgt charakterisiert: 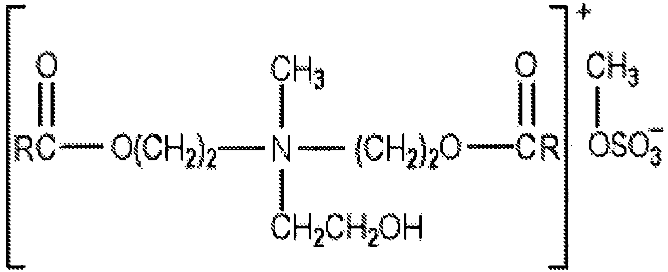 hierbei stellt R einen Palmitylrest dar.

Die Ölphase kann beispielsweise ausgewählt sein aus der Gruppe der polaren Öle, umfassend Lecithine, Fettsäuretriglyceride, Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können ausgewählt sein aus der Gruppe der synthetischen, halbsynthetischen und/oder natürlichen Öle, umfassend Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl und dergleichen.

Polare Ölkomponenten können ausgewählt werden aus der Gruppe der Ester, umfassend gesättigte und/oder ungesättigte, verzweigte und/oder unverzweigte Alkancarbonsäuren mit einer Kettenlänge von 3 bis 30 C-Atomen; gesättigte und/oder ungesättigte, verzweigte und/oder unverzweigte Alkohole mit einer Kettenlänge von 3 bis 30 C-Atomen; Ester aus aromatischen Carbonsäuren; gesättigte und/oder ungesättigte, verzweigte und/oder unverzweigte Alkohole einer Kettenlänge von 3 bis 30 C-Atomen.

Bevorzugte Esteröle umfassen Octylpalmitat, Octylcocoat, Octylisostearat, Octyldodecylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, 3-Oleyloleat, Oleylerucat, Erucyloleat, Erucylerucat, Tridecylstearat, Tridecyltrimellitat, sowie synthetische, halbsynthetische und natürliche Gemische solcher Ester, insbesondere Jojobaöl.

Die Ölphase kann aber auch Dialkylether und Dialkylcarbonate, Dicaprylylether und/oder Dicaprylylcarbonat, umfassen. Geeignete Ölkomponenten können ausgewählt sein aus der Gruppe umfassend Isoeikosan, Neopentylglykoldiheptanoat, Propylenglykoldicaprylat/Dicaprat, Caprylic/Capric/Diglyceryl-Succinat, Butylenglykol Dicaprylat/Dicaprat, C₁₂₋₁₃-Alkyllactat, Di-C₁₂₋₁₃-Alkyltartrat, Triisostearin, Dipentaerythrityl-Hexacaprylat/Hexacaprat, Propylenglykolmonoisostearat, Tricaprylin, Dimethylisosorbid, C₁₂₋₁₅-Alkylbenzoat, Butyloctylsalicylat, Hexadecylbenzoat und Butyloctylbenzoat und Gemische davon.

Als Ölphase lassen sich auch unpolare Öle verwenden, umfassend verzweigte und unverzweigte Kohlenwasserstoffe und -wachse, insbesondere Mineralöl, Vaseline, Paraffinöl, Squalan und Squalen, Polyolefine, hydrogenierte Polyisobutene und Isohexadekan.

Die Ölphase kann ferner einen Gehalt an cyclischen oder linearen Siliconölen aufweisen oder vollständig aus solchen Ölen bestehen. Systematisch werden die Siliconöle als Polyorganosiloxane bezeichnet. Die methylsubstituierten Polyorganosiloxane, welche die mengenmäßig bedeutendsten Verbindungen dieser Gruppe darstellen, werden auch als Polydimethylsiloxan bzw. Dimethicone (INCI) bezeichnet. Dimethicone gibt es in verschiedenen Kettenlängen bzw. mit verschiedenen Molekulargewichten. Geeignete Polyorganosiloxane gemäß der vorliegenden Erfindung umfassen Dimethylpolysiloxane, welche beispielsweise unter den Handelsbezeichnungen Abil 10 bis 10000 bei der Firma Goldschmidt GmbH erhältlich sind, Cetyldimethicone, Hexamethylcyclotrisiloxan, Polydimethylsiloxan, Poly(methylphenylsiloxan), Phenylmethylpolysiloxan (INCI: Phenyl Dimethicone, Phenyl Trimethicone), cyclische Silicone (Octamethylcyclotetrasiloxan bzw. Decamethylcyclopentasiloxan), welche nach INCI auch als Cyclomethicone bezeichnet werden, aminomodifizierte Silicone (INCI: Amodimethicone) und Siliconwachse, z. B. Polysiloxan-Polyalkylen-Copolymere (INCI: Stearyl Dimethicone und Cetyl Dimethicone) und Dialkoxydimethylpolysiloxane (Stearoxy Dimethicone), welche als verschiedene Abil Wax-Typen bei der Firma Goldschmidt GmbH erhältlich sind.

Insbesondere für den Fall, dass es sich bei der Zusammensetzung um ein Sonnenschutzmittel handelt, zählen auch organische Sonnenfiltersubstanzen wie z.B. Zimtsäurederivate; Salicylsäurederivate; Campherderivate; Triazinderivate; Benzophenonderivate; Dibenzoylmethanderivate; Diphenylacrylatderivate; Benzimidazolderivate; p-Aminobenzoesäurederivate, Polymerfilter und Siliconfilter; genauer:
p-Aminobenzoesäure, ethoxyliertes p-Aminobenzoat, 2-Ethylhexylp-dimethylaminobenzoat, N-propoxyliertes Ethyl-p-aminobenzoat, Glyceryl-p-aminobenzoat, Homomenthylsalicylat, 2-Ethylhexylsalicylat, Triethanolaminsalicylat, 4-Isopropylbenzylsalicylat, 4-tert.-Butyl-4'-methoxydibenzoylmethan, 4-Isopropyldibenzoylmethan, 2-Ethylhexyl-4-methoxycinnamat, Methyldiisopropylcinnamat, Isoamyl-4-methoxycinnamat, Diethanolamin-4-methoxycinnamat, Menthylanthranilat, 2-Ethylhexyl-2-cyano-3,3'-di-phenylacrylat, Ethyl-2-cyano-3,3'-diphenylacrylat, 2-, 3-(4'-Trimethylammonium)-benzyliden-bornan-2-on-methylsulfat, Urocansäure, 2-Hydroxy-4-methoxybenzophenon, 2-Hydroxy-4-methoxy-benzophenon-5-sulfonat, 2,4-Dihydroxybenzophenon, 2,2', 4,4'-Tetrahydroxybenzophenon, 2, 2'-Dihydroxy-4,4'-dimethoxybenzophenon, 2-Hydroxy-4-n-octoxybenzophenon, 2-Hydroxy-4-methoxy-4'-methylbenzophenon, 3-(4'-Methylbenzyliden)-d 1-campher, 3-Benzyliden-d 1-campher, 2,4,6-Tris-[p-(2'-ethylhexyl-1'-oxycarbonyl)-anilino]-1,3,5-triazin, 2-[p-(tert.-Butylamido)-anilino]-4,6-bis-[(p-(2'-ethylhexyl-1'-oxycarbonyl)-anilino]-1, 3,5-triazin, Polymer von N-[(2 und 4)-[(2-oxoborn-3-yliden)-methyl]-benzyl]-acrylamid, 2-[4-(Diethylamino)2-hydroxybenzoyl] alkylbenzoate, Polyorganosiloxane mit Malonatgruppe, zu den Ölen und ölartigen Bestandteilen im Sinne der obigen Definition.

Bevorzugt wird z.B. Polyethylenglykoldistearat mit einem Molekulargewicht von 6.000, das z.B. von der Firma Goldschmidt GmbH unter dem Handelsnamen REWOPAL PEG 6000 DS angeboten wird, verwendet. Letzteres wird in einer Produktinformation als Verdickungsmittel für Shampoos spezifiziert. Dabei werden Konzentrationen von 0,5 bis 5 Gew.-% empfohlen.

Weitere, einsetzbare Derivate im obigen Sinn umfassen z.B. PEG-150 Dibehenat, PEG-90 Diisostearat, PEG-175 Diisostearat, PEG-32 Dilaurat, PEG-75 Dilaurat, PEG-150 Dilaurat, PEG-32 Dioleat, PEG-75 Dioleat, PEG-150 Dioleat, PEG-30 Dipolyhydroxystearat, PEG-20 Diricinoleat, PEG-32 Distearat, PEG-75 Distearat, PEG-120 Distearat, PEG-150 Distearat, PEG-175 Distearat, PEG-190 Distearat, PEG-250 Distearat, PEG/PPG-32/3 Diricinoleat und PPG-30 Dioleat.

Besonders bevorzugt ist vorgesehen, dass die Zusammensetzung außerdem einen kosmetischen Hilfsstoff und/oder Zusatzstoff enthält, der vorzugsweise unter den Fettsubstanzen, organischen Lösemitteln, Verdickungsmitteln, reizlindernden Mitteln, Antioxidantien, Trübungsmitteln, Stabilisierungsmitteln, Emollientien, Hydroxysäuren, Antischaummitteln, Hydratisierungsmitteln, Vitaminen, Parfüms, Konservierungsmitteln, grenzflächenaktiven Stoffen, Füllstoffen, Maskierungsmitteln, Polymeren, Treibmitteln, alkalisch machenden Mitteln oder Säuerungsmitteln, und/oder Farbmitteln ausgewählt ist.

Die erwähnten Hilfs- oder Zusatzstoffe umfassen insbesondere Konservierungsmittel, Konservierungshelfer, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Füllstoffe, die das Hautgefühl verbessern, Fette, Öle, Wachse und/oder andere übliche kosmetische Hilfstoffe einer kosmetischen Formulierung umfassend Alkohole, Polyole, Polymere, Schaumstabilisatoren, Elektrolyte, organische Lösungsmittel und/oder Siliconderivate. Erfindungsgemäß bevorzugt verwendbare kosmetische Hilfsstoffe umfassen Alkohole, Ethanol und/oder Isopropanol, Diole oder Polyole sowie deren Ether, insbesondere Propylenglykol, Glycerin, Ethylenglykol, Ethylenglykolmonoethyl- oder -monobutylether, Propylenglykolmonomethyl-, -monoethyl- oder -monobutylether, Diethylenglykolmonomethyl- oder -monoethylether und analoge Produkte, Polymere, Schaumstabilisatoren, Elektrolyte, Dihydoxyaceton sowie gegebenenfalls ein oder mehrere Verdickungsmittel, wie Siliciumdioxid, Aluminiumsilikate, Polysaccharide sowie Derivate davon, Hyaluronsäure, Xanthangummi, Hydroxypropylmethylcellulose, Polyacrylate, Carbopole, beispielsweise Carbopole der Typen 980, 981, 1382, 2984, 5984, jeweils einzeln oder in Kombination.

Geeignete verwendbare kosmetische Hilfsstoffe sind beispielsweise in der EP-A-1 566 170 beschrieben, auf die hier in vollem Umfang Bezug genommen wird.

Die kosmetische Zusammensetzung der vorliegenden Erfindung kann auch Moisturizer aufweisen. Als Moisturizer werden Stoffe oder Stoffgemische bezeichnet, welche kosmetischen Zusammensetzungen die Eigenschaft verleihen, nach dem Auftragen bzw. Verteilen auf der Hautoberfläche die Feuchtigkeitsabgabe der Hornschicht zu reduzieren und/oder die Hydratation der Hornschicht positiv zu beeinflussen. Verwendbare Moisturizer umfassen Glycerin, Milchsäure und/oder Lactate, insbesondere Natriumlactat, Butylenglykol, Propylenglykol, Biosaccaride, Glycine, Soja, Ethylhexyloxyglycerin, Pyrrolidoncarbonsäure und Harnstoff, sowie polymere Moisturizer aus der Gruppe der wasserlöslichen und/oder in Wasser quellbaren und/oder mit Hilfe von Wasser gelierbaren Polysaccharide. Geeignet sind ferner Hyaluronsäure, Chitosan und/oder ein fucosereiches Polysaccharid, welches in den Chemical Abstracts unter der Registraturnummer 178463-23-135 abgelegt und beispielsweise unter der Bezeichnung Fucogel 1000 von der Gesellschaft SOLABIA S.A., erhältlich ist.

Verwendbare Konservierungsmittel im Sinne der vorliegenden Erfindung umfassen Formaldehydabspalter, Iodopropinylbutylcarbamate, Paraben, p-Hydroxybenzoesäurealkylester, wie Methyl-, Ethyl-, Propyl- und/oder Butylparaben, Phenoxyethanol, Ethanol, Benzoesäure. Üblicherweise umfasst das Konservierungssystem erfindungsgemäß ferner vorteilhaft auch Konservierungshelfer, wie beispielsweise Octoxyglycerin, Glycin, und/oder Soja.

Verwendbare Antioxidatien im Sinne der vorliegenden Erfindung umfassen wasserlösliche Antioxidantien, Vitamine, insbesondere Ascorbinsäure und deren Derivate. Bevorzugte Antioxidantien sind ferner Vitamin E und dessen Derivate sowie Vitamin A und dessen Derivate. Die Menge der Antioxidantien, bezogen auf das Gesamtgewicht der kosmetischen Zusammensetzung kann 0,001 bis 30 Gew.-%, bevorzugt 0,05 bis 15 Gew.-%, insbesondere 0,5 bis 10 Gew.-%, bezogen auf das Gesamtgewicht der Zusammensetzung, ausmachen.
Verwendbare natürliche Wirkstoffe und/oder deren Derivate umfassen alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin. Hinzukommen sekundäre Pflanzenstoffe mit antioxidativen Eigenschaften, insbesondere Polyphenole und deren Derivate.

Die kosmetische Zusammensetzung kann auch Füllstoffe enthalten, welche die sensorischen und kosmetischen Eigenschaften der Formulierungen weiter verbessern und beispielsweise ein samtiges oder seidiges Hautgefühl hervorrufen oder verstärken. Verwendbare Füllstoffe umfassen Stärke und Stärkederivate, wie Tapiocastärke, Distärkephosphat, Aluminium- bzw. Natrium-Stärke -Octenylsuccinat, sowie Pigmente, die weder hauptsächlich UV-Filter- noch färbende Wirkung haben, beispielsweise Bornitrid und/oder Aerosile (CAS-Nr. 7631-86-9).

Ferner kann es gegebenenfalls von Vorteil sein, Filmbildner in die erfindungsgemäßen kosmetischen Zusammensetzungen einzuarbeiten, beispielsweise um die Wasserfestigkeit der Zusammensetzungen zu verbessern oder die UV-Schutzleistung zu erhöhen. Geeignet sind sowohl wasserlösliche, dispergierbare und/oder fettlösliche Filmbildner, jeweils einzeln oder in Kombination miteinander. Erfindungsgemäß verwendbare Filmbildner umfassen Polyurethane, Polyisobutene, hydrogenierte Polyisobutene, Dimethicone Copolyol, Polyacrylate, PVP/VA Copolymere (PVP = Polyvinylpyrrolidon, VA = Vinylacetat).

Fettlösliche Filmbildner können ausgewählt sein aus der Gruppe der Polymere auf Basis von Polyvinylpyrrolidon (PVP), Copolymere des Polyvinylpyrrolidons, PVP/Hexadecen-Copolymer und/oder das PVP/Eicosen-Copolymer.

Weiterhin kann die erfindungsgemäße kosmetische Zusammensetzung außerdem ein oder mehrere ergänzende hydrophile oder lipophile, im UV-A- und/oder UV-B-Bereich wirksame organische Filter, und/oder Pigmente oder Nanopigmente von Metalloxiden, die umhüllt oder nicht umhüllt sind, enthalten. Hierbei kann es sich z.B. um Octocrylen, Octylmethoxycinnamat oder Butylmethoxydibenzoylmethan handeln. Weitere öllösliche UVB- und/oder Breitband-Filtersubstanzen im Sinne der vorliegenden Erfindung umfassen 3-Benzylidencampher-Derivate, vorzugsweise 3-(4-Methylbenzyliden)campher, 3-Benzylidencampher-4-i3-Aminobenzoesaure-Derivate, vorzugsweise 4-(Dimethylamino)-benzoesäure(2-ethylhexyl)ester, 4-Dimethylamino-benzoesäureamylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie an Polymere gebundene UV-Filter, wie 3-(4-(2,2-bis-Ethoxycarbonylvinyl)-phenoxy)propenyl)-methoxysiloxan/Dimethylsiloxan-Copolymer.

Ferner können die erfindungsgemäßen kosmetischen Zusammensetzungen Pigmente oder auch Nanopigmente mit Partikelgrößen im Bereich von 5 bis 200 nm, vorzugsweise im Bereich von 10 bis 50 nm, die umhüllt oder nicht umhüllt sind, enthalten. Geeignet verwendbare Nanopigmente von Metalloxiden umfassen Titandioxid, beispielsweise amorph oder kristallin in Form von Rutil und/oder Anatas, Eisen, Mangan, Zink, Zirconium oder Cer, welche durchweg bekannte UV-Lichtschutzsubstanzen darstellen.

Herkömmliche Umhüllungsmittel können Aluminiumoxid, Siliciumdioxid, Aluminiumstearat, Trimethoxycaprylylsilane, Triethoxycaprylylsilane, Dimethicone und/oder Methicone sein. Erfindungsgemäß verwendbare Nanopigmente von Metalloxiden, die gegebenenfalls umhüllt sind, werden beispielsweise in den Patentanmeldungen EP-A-0 518 772 und EP-A-0 518 773 beschrieben. Erfindungsgemäß verwendbare kosmetische Zusammensetzungen können 0,1 bis 20 Gew.-%, vorzugsweise 0,5 bis 15 Gew.-%, und bevorzugt 0,5 bis 10 Gew.-% einer oder mehrerer dieser UV-Filtersubstanzen aufweisen. Überdies ist erfindungsgemäß in einer bevorzugten Ausgestaltung vorgesehen, dass die Zusammensetzung außerdem mindestens ein Mittel zum bräunen und/oder künstlichen braunfärben der Haut enthält. Hierbei kann es sich z.B. um Dihydroxyaceton handeln.

Bei der kosmetischen Zusammensetzung handelt es sich bevorzugt um eine Zusammensetzung zur Pflege oder zum Schutz der menschlichen Epidermis oder um ein Sonnenschutzmittel, die oder das in Form einer nicht-ionischen Vesikeldispersion, einer Emulsion, insbesondere einer Emulsion vom Öl-in-Wasser-Typ, einer Milch, eines Gels, einer Dispersion oder eines Sprays vorliegt.

In diesem Zusammenhang kommen vor allem Körperlotionen, Selbstbräuner, Sonnenschutzmittel, After-Sun-Lotionen und dergleichen in Betracht.

Weiterhin ist erfindungsgemäß ein Verfahren zur topischen kosmetischen Behandlung der Haut zwecks Erzielung einer pflegenden oder schützenden Wirkung vorgesehen, das dadurch gekennzeichnet ist, dass es darin besteht, auf die Haut eine wirksame Menge einer wie oben definierten Zusammensetzung aufzutragen.

Beispiele:
Die vorliegende Erfindung wird durch die im Folgenden diskutierten Beispiele genauer erläutert. Dabei ist zu beachten, dass die Beispiele nur beschreibenden Charakter haben und nicht dazu gedacht sind, die Erfindung in irgendeiner Form einzuschränken.
Die in den folgenden Tabellen aufgeführten Komponenten der Phase A bzw. der Phase B werden getrennt auf 70 °C aufgeheizt, miteinander verrührt, homogenisiert und bis auf 40 °C kalt gerührt. Anschließend wird ein Konservierungsmittel zugesetzt, das Produkt wird auf 30 °C kalt gerührt, gegebenenfalls Phase C eingerührt und dann abgefüllt. Nach 3 Tagen wird die Viskosität ermittelt. Phase A ist dabei die Phase, die die Öle und ölartigen Bestandteile enthält.

**Tabelle 1:**

| Bestandteil | Gew.-% | | | | |
|---|---|---|---|---|---|
| **Phase A** | | | | | |
| REWOQUAT® WE 38 | 3,5 | 3,5 | 3,5 | 3,5 | 3,5 |
| TEGIN® M | 1,5 | 1,5 | 1,5 | 1,5 | 1,5 |
| TEGO® Alkanol 18 | 1,0 | 1,0 | 1,0 | 1,0 | 1,0 |
| Mineralöl | 1,5 | 1,5 | 1,5 | 1,5 | 1,5 |
| TEGOSOFT® CT | 5,0 | 5,0 | 5,0 | 5,0 | 5,0 |
| ABIL® 100 | 1,5 | 1,5 | 1,5 | 1,5 | 1,5 |
| TEGOSOFT® P | 1,0 | 1,0 | 1,0 | 1,0 | 1,0 |
| OMC | 5,0 | 5,0 | 5,0 | 5,0 | 5,0 |
| OC | 3,0 | 3,0 | 3,0 | 3,0 | 3,0 |
| BMDM | 2,5 | 2,5 | 2,5 | 2,5 | 2,5 |

| **Phase B** | | | | | |
|---|---|---|---|---|---|
| Glycerin | 3,0 | 3,0 | 3,0 | 3,0 | 3,0 |
| Harnstoff | 0,5 | 0,5 | 0,5 | 0,5 | 0,5 |
| Wasser | 69,38 | 68,88 | 68,38 | 67,88 | 66,88 |
| **REWOPAL® PEG 6000 DS** | **0,5** | **1,0** | **1,5** | **2,0** | **3,0** |
| Na₂HPO₄ * 12H₂O (20%-ige Lsg.) | 0,12 | 0,12 | 0,12 | 0,12 | 0,12 |
| Phenonip® | 1,0 | 1,0 | 1,0 | 1,0 | 1,0 |
| 3 Tage | | | | | |
| pH-Wert | 4,5 | 4,3 | 4,1 | 3,9 | 3,6 |
| Viskosität (5/10) * mPas | 60 | 80 | 120 | 240 | 560 |

| | | | | | |
|---|---|---|---|---|---|
| *Viskositätsmessung mit Brookfield RVT, Spindel Nr. 5, 10 Upm | | | | | |

**Tabelle 2:**

| Bestandteil | Gew.-% | | | | |
|---|---|---|---|---|---|
| **Phase A** | | | | | |
| REWOQUAT® WE 38 | 3,5 | 3,5 | 3,5 | 3,5 | 3,5 |
| TEGIN® M | 1,5 | 1,5 | 1,5 | 1,5 | 1,5 |
| TEGO® Alkanol 18 | 1,0 | 1,0 | 1,0 | 1,0 | 1,0 |
| Mineralöl | 1,5 | 1,5 | 1,5 | 1,5 | 1,5 |
| TEGOSOFT® CT | 5,0 | 5,0 | 5,0 | 5,0 | 5,0 |
| ABIL® 100 | 1,5 | 1,5 | 1,5 | 1,5 | 1,5 |
| TEGOSOFT® P | 1,0 | 1,0 | 1,0 | 1,0 | 1,0 |
| OMC | 5,0 | 5,0 | 5,0 | 5,0 | 5,0 |
| OC | 3,0 | 3,0 | 3,0 | 3,0 | 3,0 |
| BMDM | 2,5 | 2,5 | 2,5 | 2,5 | 2,5 |

| **Phase B** | | | | | |
|---|---|---|---|---|---|
| Glycerin | 3,0 | 3,0 | 3,0 | 3,0 | 3,0 |
| Harnstoff | 0,5 | 0,5 | 0,5 | 0,5 | 0,5 |
| Wasser | 69,68 | 69,38 | 68,88 | 68,38 | 67,88 |
| **AMILAN® Guar 39** | **0,2** | **0,5** | **1,0** | **1,5** | **2,0** |
| Na₂HPO₄ * 12H₂O (20%-ige Lsg.) | 0,12 | 0,12 | 0,12 | 0,12 | 0,12 |
| Phenonip® | 1,0 | 1,0 | 1,0 | 1,0 | 1,0 |
| 3 Tage | | | | | |
| pH-Wert | 5,8 | 5,8 | 6,1 | 6,5 | |
| Viskosität (5/10)* mPas | 3400 | 4300 | 15400 | 39600 | n/n |

| | | | | | |
|---|---|---|---|---|---|
| *Viskositätsmessung mit Brookfield RVT, Spindel Nr. 5, 10 Upm | | | | | |

**Tabelle 3:**

| Bestandteil | Gew.-% | | |
|---|---|---|---|
| **Phase A** | | | |
| REWOQUAT® WE 38 | 3,5 | ---- | ---- |
| FS Palm 1850 H | ---- | 3,5 | ---- |
| VARISOFT® EQ 65 | ---- | ---- | 3,5 |
| TEGO® Alkanol 18 | 1,0 | ---- | ---- |
| TEGIN® M 1,5 | 1,5 | 1,5 | 1,5 |
| TEGOSOFT® CR | 1,5 | 1,5 | 1,5 |
| TEGOSOFT® TN | 5,0 | 6,0 | 6,0 |
| TEGOSOFT® E | 1,5 | 1,5 | 1,5 |
| TEGOSOFT® TIS | 1,0 | 1,0 | 1,0 |
| OMC | 5, 0 | 5, 0 | 5, 0 |
| OC | 3,0 | 3,0 | 3,0 |
| BMDM | 2, 5 | 2, 5 | 2, 5 |

| **Phase B** | | | |
|---|---|---|---|
| Glycerin | 3,0 | 3,0 | 3,0 |
| Harnstoff | 0,5 | 0,5 | 0,5 |
| **AMILAN® Guar 39** | **0,5** | **0,5** | **0,5** |
| **REWOPAL® PEG 6000 DS** | **0,2** | **0,2** | **0,2** |
| Na₂HPO₄ * 12H₂O (20%-ige Lsg) | 1,5 | 1,5 | 1,5 |
| Wasser | 67,8 | 67,8 | 67,8 |
| Phenonip® | 1,0 | 1,0 | 1,0 |
| 3 Tage | | | |
| pH-Wert | 5,1 | 4,9 | 5,2 |
| Viskosität (5/10)* mPas | 3900 | 3600 | 9600 |

| | | | |
|---|---|---|---|
| *Viskositätsmessung mit Brookfield RVT, Spindel Nr. 5, 10 Upm | | | |

**Tabelle 4:**

| Bestandteil | Gew.-% | |
|---|---|---|
| **Phase A** | | |
| VARISOFT® TA 100 | 3,50 | 3,50 |
| TEGIN® M | 2,00 | 2,00 |
| TEGO® Alkanol 18 | 0,5 | 0,5 |
| TEGO® Care 450 | 1,50 | 1,50 |
| OC | 10,00 | 10,00 |
| TEGO® Sun TDEC 45 | 10,00 | 8,00 |
| Ethylhexyl Salicylate | | 5,00 |
| TEGOSOFT® TN | 4,00 | 4, 00 |
| Vitamin-E-acetat | 0,10 | 0,10 |
| **Polyquaternium-37** | **0,30** | **0,30** |
| BMDM | 3,00 | 3,00 |

| **Phase B** | | |
|---|---|---|
| Glycerin 99,5 %ig | 3,00 | 3,00 |
| Wasser | 61,00 | 58,00 |
| TEGO® Cosmo C 100 (Creatine) | 0,10 | 0,10 |
| Allantoin gepulvert | 0,10 | 0,10 |

| **Phase C** | | |
|---|---|---|
| Symdiol 68 T (1,2-Hexanediol) | 0,70 | 0,70 |
| Parfum | 0,20 | 0,20 |
| 3 Tage | | |
| pH-Wert | 4,7 | 4,8 |
| Viskosität (5/10)* mPas | 10.000 | 9.500 |

| | | |
|---|---|---|
| *Viskositätsmessung mit Brookfield RVT, Spindel Nr. 5, 10 Upm | | |

Bei den verwendeten Produkten handelt es sich um folgende Substanzen:
- REWOQUAT^{®} WE 38:: Esterquat, erhältlich bei der Firma Goldschmidt GmbH,
- TEGIN^{®} M:: Glycerin-Monostearat, erhältlich bei der Firma Goldschmidt GmbH,
- TEGO^{®} Alkanol 18:: Stearylalkohol, erhältlich bei der Firma Goldschmidt GmbH,
- TEGOSOFT^{®} CT:: Caprylic/Capric Triglyceride, erhältlich bei der Firma Goldschmidt GmbH,
- TEGO^{®} Sun TDEC 45:: hochstabile Dispersion aus Titandioxid, Diethylhexyl Carbonat und Polyglyceryl-6 Polyhydroxystearat, erhältlich bei der Firma Goldschmidt GmbH,
- TEGO^{®} Care 450:: Polyglyceryl-3 Methylglucose Distearat, erhältlich bei der Firma Goldschmidt GmbH,
- ABIL^{®} 100:: Dimethicone, erhältlich bei der Firma Goldschmidt GmbH,
- TEGOSOFT^{®} P:: Isopropylpalmitat, erhältlich bei der Firma Goldschmidt GmbH,
- OMC:: Ethylhexylmethoxycinnamat,
- OC:: Octocrylen,
- BMDM:: Butylmethoxydibenzoylmethan,
- REWOPAL^{®} PEG 6000 DS:: PEG-150 Distearate, erhältlich bei der Firma Goldschmidt GmbH,
- Phenonip:: Konservierungsmittel, enthaltend Phenoxyethanol, Methylparaben, Butylparaben, Ethylparaben und Propylparaben, erhältlich bei der Firma Clariant UK Ltd,
- AMILAN^{®} Guar 39:: quaternisiertes Guar, erhältlich bei der Firma Goldschmidt GmbH,
- VARISOFT® EQ 65:: Esterquat aus Methyldiethanolamine mit Stearinsäure und 35 % Cetearylalkohol, erhältlich bei der Firma Goldschmidt GmbH,
- TEGOSOFT® TIS:: Triisostearin, erhältlich bei der Firma Goldschmidt GmbH,
- TEGOSOFT® E:: PPG-15 Stearyl Ether, erhältlich bei der Firma Goldschmidt GmbH,
- TEGOSOFT® TN:: C₁₂₋₁₅-Alkyl-benzoat, erhältlich bei der Firma Goldschmidt GmbH,
- TEGOSOFT® CR:: Cetyl-Ricinoleat, erhältlich bei der Firma Goldschmidt GmbH,
- FS Palm 1850 H:: Dipalmoylethyl-Esterquat und Stearylalkohol,
- VARISOFT® TA 100: Distearyldimethylammonium Chlorid, erhältlich bei der Firma Goldschmidt GmbH,
- Polyquaternium-37:: Poly-N,N,N-trimethyl-2-((2-methyl-1-oxo-2-propenyl) oxy) chlorid,
- TEGO® Cosmo C 100:: Creatin, erhältlich bei der Firma Goldschmidt GmbH.

Die in der Tabelle mit ® gekennzeichneten Produktbezeichnungen sind eingetragene Warenzeichen.

In Tabelle 1 sind Zusammensetzungen gezeigt, die neben dem Esterquat REWOQUAT® WE 38 Polyethylenglykol-Distearat in verschiedenen Konzentrationen enthalten. In Tabelle 2 sind Zusammensetzungen gezeigt, die neben dem Esterquat REWOQUAT® WE 38 das quaternisierte Polysaccharid AMILAN® Guar 39 in verschiedenen Konzentrationen enthalten. In beiden Fällen bilden die Bestandteile Mineralöl, TEGOSOFT® CT, ABIL® 100 und TEGOSOFT® P, OMC, OC und BMDM die öl- bzw. ölartige Fraktion, deren Gesamtanteil bei 16 Gew.-% liegt.

Es ist klar erkennbar, das sich bei Verwendung von 0,5 bis 3 Gew.-% Polyethylenglykol-Distearat sehr niedrige Viskositäten einstellen, die innerhalb des beanspruchten Bereichs liegen. Dies ist überraschend, da Polyethylenglykol-Distearat eigentlich als Verdickungsmittel fungiert. Gleichwohl wird gegenüber einer vergleichbaren Zusammensetzung ohne Polyethylenglykol-Distearat eine wesentliche Stabilitätsverbesserung erzielt.

Es ist ebenso klar erkennbar, das sich bei Verwendung von 0,2 oder 0,5 Gew.-% Amilan Guar 39 eine Anfangsviskosität von 4.300 mPas einstellt. Auch dieser Befund ist überraschend, da Amilan Guar 39 ebenfalls als Verdickungsmittel fungiert. Gleichwohl wird gegenüber einer vergleichbaren Zusammensetzung ohne Amilan Guar 39 eine wesentliche Stabilitätsverbesserung erzielt. Höhere Konzentrationen, die außerhalb des beanspruchten Bereichs liegen, führen hingegen zu Viskositäten, die außerhalb des beanspruchten Viskositätsbereichs liegen.

In Tabelle 3 sind Zusammensetzungen gezeigt, die neben Esterquats (REWOQUAT E 38 bzw. VARISOFT® EQ 65) sowohl Polyethylenglykol-Distearat als auch Amilan Guar 39 enthalten. Auch hier werden stabile kationische Emulsionen im geforderten Viskositätsbereich erhalten.

In Tabelle 4 sind Zusammensetzungen gezeigt, die neben dem Dialkylquat VARISOFT® TA 100 Polyquaternium-37 enthalten. Auch hier ist erkennbar, dass stabile kationische Emulsionen im geforderten Viskositätsbereich erhalten werden.

## Patentansprüche

1. Kosmetische Zusammensetzung zum Auftragen auf die Haut, mit einer Viskosität zwischen 50 und 1.000 mPas bei Raumtemperatur aufweisend
a) 0,05 bis 5,0 Gew.-% eines Polyethylen- bzw. Polypropylenglykolderivats, wobei dieses ausgewählt ist aus einem Polyethylenglykoldiester mit einem Molekulargewicht im Bereich zwischen 1.500 und 12.000 Dalton, einem Polypropylenglykoldiester oder einem Polyethylen/Polypropylenglykoldiester,
b) 10 bis 40 Gew.-% Öle und/oder ölartige Bestandteile, sowie
c) 0,2 bis 15 Gew.-% eines Esterquats.

2. Kosmetische Zusammensetzung gemäß zumindest einem der vorherigen Ansprüche, **dadurch gekennzeichnet, dass** die Zusammensetzung außerdem einen kosmetischen Hilfsstoff und/oder Zusatzstoff enthält, der unter den Fettsubstanzen, organischen Lösemitteln, Verdickungsmitteln, Antioxidantien, Trübungsmitteln, Stabilisierungsmitteln, Emollientien, Hydroxysäuren, Antischaummitteln, Hydratisierungsmitteln, Vitaminen, Parfüms, Konservierungsmitteln, grenzflachenaktiven Stoffen, Füllstoffen, Maskierungsmitteln, Polymeren, Treibmitteln, alkalisch machenden Mitteln oder Säuerungsmitteln und/oder Farbmitteln, ausgewählt ist.

3. Kosmetische Zusammensetzung gemäß zumindest einem der vorherigen Ansprüche, **dadurch gekennzeichnet, dass** sie außerdem ein oder mehrere ergänzende hydrophile oder lipophile, im UV-A- und/oder UV-B-Bereich wirksame organische Filter, und/oder Pigmente oder Nanopigmente von Metalloxiden, die umhüllt oder nicht umhüllt sind, enthält.

4. Kosmetische Zusammensetzung gemäß zumindest einem der vorherigen Ansprüche, **dadurch gekennzeichnet, dass** sie außerdem mindestens ein Mittel zum Bräunen und/oder künstlichen Braunfärben der Haut enthält.

5. Kosmetische Zusammensetzung gemäß zumindest einem der vorherigen Ansprüche, **dadurch gekennzeichnet, dass** es sich um eine Zusammensetzung zur Pflege oder zum Schutz der menschlichen Epidermis oder ein Sonnenschutzmittel handelt und dass sie in Form einer nicht-ionischen Vesikeldispersion, einer Emulsion, insbesondere einer Emulsion vom Öl-in-Wasser-Typ, einer Milch, eines Gels einer Dispersion oder eines Sprays vorliegt.

## Claims

1. Cosmetic composition for topical skin application, having a viscosity of between 50 and 1,000 mPas at room temperature, comprising
a) 0.05 to 5.0 wt-% of a polyethylene and/or polypropylene derivative, selected from the group consisting of a polyethylene glycol diester having a molecular weight of between 1,500 and 12,000 Dalton, a polypropylene glycol diester, or a polyethylene/polypropylene glycol diester,
b) between 10 and 40 wt-% of oils and/or oil-like components, and
c) between 0,2 and 15 wt-% of an esterquat.

2. Cosmetic composition according to at least one of the preceding claims, **characterized in that** said composition additionally contains a cosmetic adjuvant and/or additive selected from the group consisting of fatty substances, organic solvents, thickeners, antioxidants, opacifiers, stabilizers, emollients, hydroxy acids, anti-foaming agents, hydration agents, vitamins, fragrances, preservatives, surface-active agents, fillers, masking agents, polymers, expanding agents, alkalifying agents or acidifiers and/or colorants.

3. Cosmetic composition according to at least one of the preceding claims, **characterized in that** it furthermore contains one or plural additional hydrophilic or lipophilic organic filters which are active in the UV-A and/or UV-B ranges, and/or coated or uncoated metal oxide pigments or nanopigments.

4. Cosmetic composition according to at least one of the preceding claims, **characterized in that** it furthermore contains at least one agent for tanning and/or artificially bronzing the skin.

5. Cosmetic composition according to at least one of the preceding claims, **characterized in that** it is a composition for human skin care or protection or a sunscreen product and that it is in the form of a non-ionic vesicle dispersion, an emulsion, in particular an oil-in-water type emulsion, a lotion, a gel, a dispersion or a spray.

## Revendications

1. Composition cosmétique à appliquer sur la peau, avec une viscosité comprise entre 50 et 1 000 mPas à température ambiante, présentant
a) 0,05 à 5,0 % en poids d'un dérivé de polyéthylène ou de polypropylène glycol, dans laquelle ce dernier est choisi parmi un diester de polyéthylène glycol avec un poids moléculaire compris dans une fourchette de 1 500 à 12 000 daltons, un diester de polypropylène glycol ou un diester de polyéthylène/polypropylène glycol,
b) 10 à 40 % en poids d'huiles et/ou de composants oléagineux, ainsi que
c) 0,2 à 15 % en poids d'un ester quaternaire.

2. Composition cosmétique selon au moins l'une des revendications précédentes, **caractérisée en ce que** la composition contient en plus un additif et/ou un adjuvant cosmétique, qui est/sont choisi(s) parmi les substances grasses, les solvants organiques, les agents épaississants, les antioxydants, les agents opacifiants, les agents stabilisants, les émollients, les acides lrydroxyliques, les agents antimousse, les agents hydratants, les vitamines, les parfums, les agents de conservation, les substances tensioactives, les charges, les agents de masquage, les polymères, les agents moussants, les agents d'alcalinisation ou les agents d'acidification et/ou les colorants.

3. Composition cosmétique selon au moins l'une des revendications précédentes, **caractérisée en ce qu'**elle contient en outre un ou plusieurs filtre(s) organique(s) complémentaire(s) hydrophile(s) ou lipophile(s) actif(s) dans le domaine des UV-A et/ou des UV-B et/ou des pigments ou des nanopigments d'oxydes métalliques, qui sont enrobés ou ne sont pas enrobés.

4. Composition cosmétique selon au moins l'une des revendications précédentes, **caractérisée en ce qu'**elle contient en outre au moins un agent bronzant et/ou de bronzage artificiel de la peau.

5. Composition cosmétique selon au moins l'une des revendications précédentes, **caractérisée en ce qu'**elle constitue une composition pour le soin ou pour la protection de l'épiderme humain ou un agent de protection solaire et **en ce qu'**elle se présente sous la forme d'une dispersion vésiculaire non ionique, d'une émulsion, en particulier d'une émulsion du type huile dans l'eau, d'un lait, d'un gel, d'une dispersion ou d'un spray.
